# EUROPEAN PATENT APPLICATION

(11) **EP 1 935 808 A2**
(43) Date of publication of application: **25.06.2008**
(21) Application number: 07254836.5
(22) Date of filing: 12.12.2007
(51) Int. Cl.: B65D 83/08

(54) **Personal care product dispenser**

(30) Priority: 19.12.2006 US 612778
(71) Applicant: Johnson and Johnson Consumer Companies, Inc., Skillman, NJ 08558 (US)
(72) Inventor: Adler, Ari Tao, Cambridge MA 02139 (US); Belfort, Jonathan, Cambridge MA 02139 (US); Stewart, William Charles, Ipswich MA 01938 (US); Klein, Devorah, Cambridge MA 02138 (US); Judson, Jared Alden, Topsfield MA 01983 (US)
(74) Representative: James, Anthony Christopher W.P.

(57) **Abstract**

The present invention is directed to devices suitable for storing an assembly (40) of multiple personal care products and individually dispensing the personal care products from the assembly, where the devices include a compartment for storing the assembly of personal care products and a mechanism for dispensing the personal care products slidingly engaged with the compartment between a storage position and a dispensing position, where the mechanism for dispensing includes a member (26) for contacting the personal care products and an opening (25) through which the products may be accessed.

## Description

### Field of the Invention

The present invention relates to devices suitable for storing an assembly of multiple personal care products and individually dispensing the personal care products from the assembly contained within the device.

### Background of the Invention

Personal care products such as adhesive bandages and patches, sanitary pads, panty liners, cleansing and cosmetic wipes and the like, may be packaged in bulk within boxes or plastic bags. In some cases, individual products contained within the packaging are contained within their own envelope, or cover, so as to require removal of the envelope prior to use by the consumer. Such bulk packaging may become damaged during storage by the consumer, in which case the individual products may become disorganized or damaged, such that they are lost or discarded prior to being used. Additionally, in order to carry multiple products in, for instance, a vehicle or personal handbag, or to maintain a supply of products in, for instance, ones office or place of employment, one either must carry the entire package of products, or carry and store multiple products loosely and unprotected. This again may lead to loss or damage of product prior to use.

United States Patent Application Publication No. US 2005/0133524 A1 describes a device for packaging and dispensing stacked items, e.g. cosmetic wipes, which device includes a box that contains a first portion, e.g. a cover, and a second portion, e.g. a drawer, that is movable relative to the first portion between a retracted position and an extended position, and a stack of items disposed in the second portion. The first portion includes a drive member configured to press against the stack of items while the second portion is being moved toward the retracted position and to entrain an item in contact with the drive member to a remainder of the stack in order to enable the item to be grasped by a user.

In devices disclosed in US 2005/0133524 A1, the drawer comprises first and second hinged portions interconnected by a hinge. The drawer thus may be in an opened position for loading the products into the drawer, and a closed position when placed in the cover for use. The first portion includes a bottom wall and a front wall that slopes upwards and forwards to form a ramp. The second portion comprises a frame and a rear wall which, when the drawer is in the closed position inside the cover, extends rearwards and downwards, at an angle of inclination relative to the bottom wall which is substantially the same as the angle of inclination of the ramp. When in the closed position inside the cover, the relative position of the first and second portions form an opening in the second portion through which the products may be directed by the drive member of the cover.

While such devices permit storage and dispensing of such products, the construction, operation and methods of making such devices are complex. In addition, once driven through the slot in the second portion by the drive member for access by the user, there is no apparent way that the devices would permit a product to be returned to the drawer for subsequent use in the event the user elects not to use the product at that time, thus resulting in wasted product.

There thus is a need for improved packaging devices suitable for storing and dispensing personal care products that provide not only more convenient and efficient storage of the products, but also that aid in dispensing the personal care products contained within the improved packaging. There also is a need for improved products that are suitable for use in such devices. Devices of the present invention provide such advantages by providing improved compartments for storage of multiple personal care products, improved, convenient and efficient means for dispensing individual products from the storage compartments and improved products suitable for use in such devices.

### Summary of the Invention

The present invention is directed to devices suitable for storing and dispensing personal care products, each personal care product of which is placed directly upon the other so as to provide an assembly comprising a plurality of personal care products. The devices include a compartment for storing the assembly of the plurality of personal care products and means for dispensing the personal care products. The plurality of personal care products includes a first personal care product, a second personal care product and multiple other personal care products disposed between the first and second personal care products. The means for dispensing the personal care product includes a member for contacting the first personal care product and an opening for receiving the first personal care product once contacted by the dispensing member. The first personal care product is located proximate the dispensing means such that it may be contacted and dispensed by the dispensing means upon operation of the device.

The storage compartment and the means for dispensing are slidingly engaged between a first storage position and a second dispensing position. When the assembly is disposed within the compartment and upon movement of the device from the first storage position to the second dispensing position, a first portion of the first personal care product is contacted by the dispensing member and directed to the opening of the dispensing means, such that the product may be accessed and easily removed from the device by the consumer. In certain embodiments, one may return the device to the storage position without having removed the exposed first personal care product, thus leaving the first personal care product in place for removal at a later time. This is an advantage in avoiding wasted product where the consumer may unintentionally expose the first product, or it is determined after exposing the first product that it is not needed at the time.

### Brief Description of the Drawings

Figure 1A is a perspective view of a device according to the present invention in a closed position.
Figure 2 is a perspective view of the device in Figure 1 shown in an open position and depicting an assembly of personal care products in phantom disposed within the device.

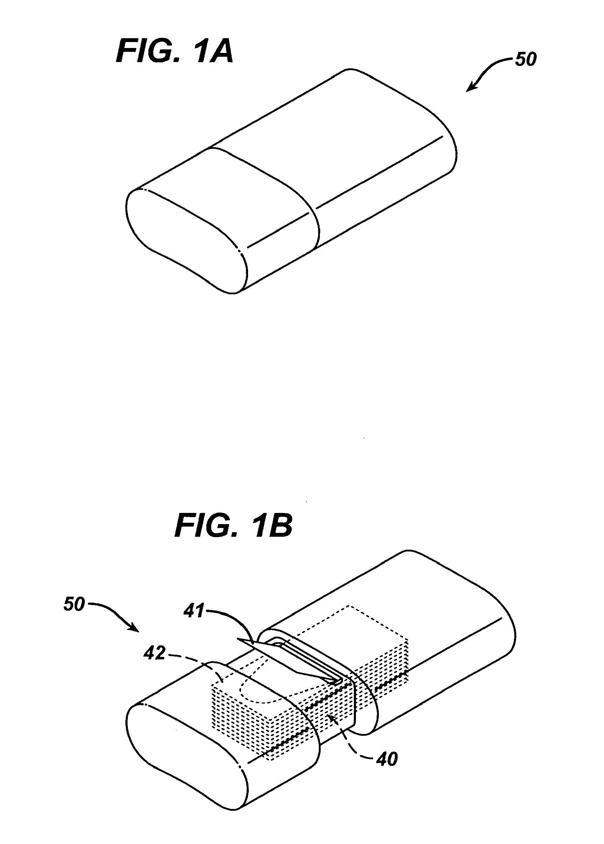

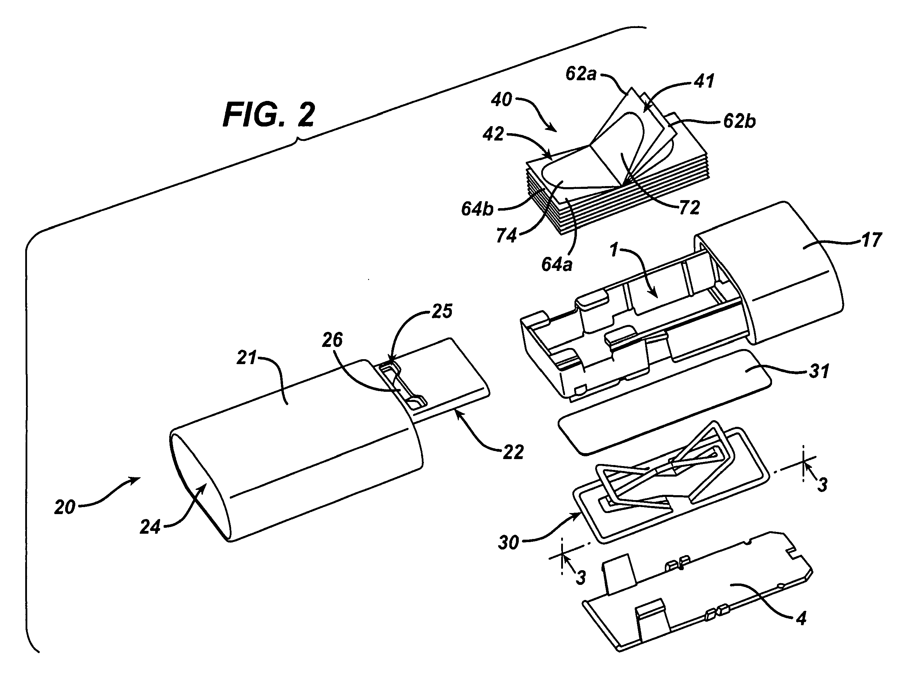

Figure 2 is an exploded perspective view of the device of Figures 1A and 1B.

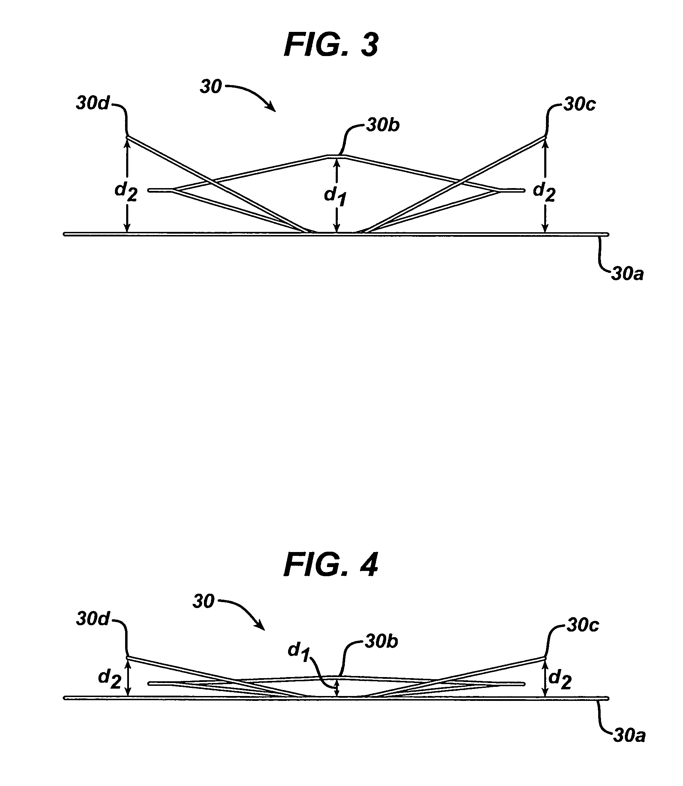

Figure 3 is a side view of advancing means 30 of Figure 2.
Figure 4 is a side view of the advancing means in Figure 3 shown in a compressed position.

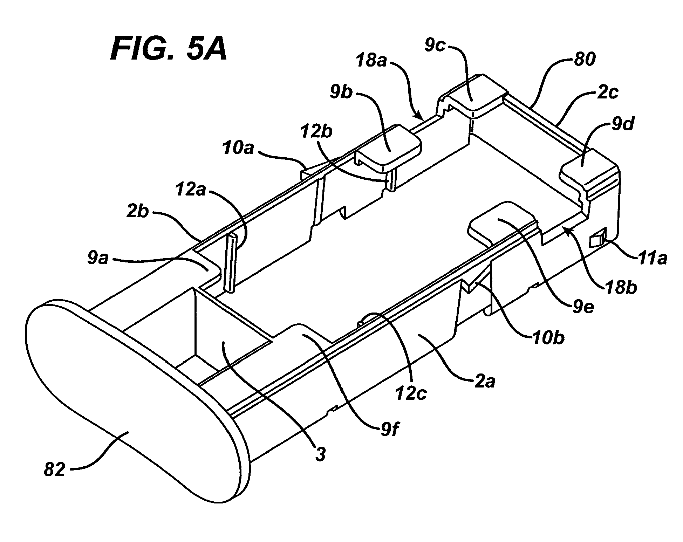

Figure 5A is a top view of a cartridge used in devices according to the present invention.

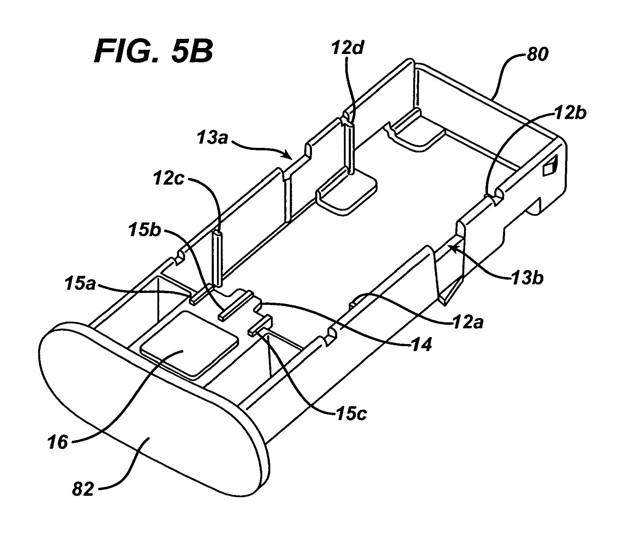

Figure 5B is a bottom view of the cartridge of Fig. 5A.

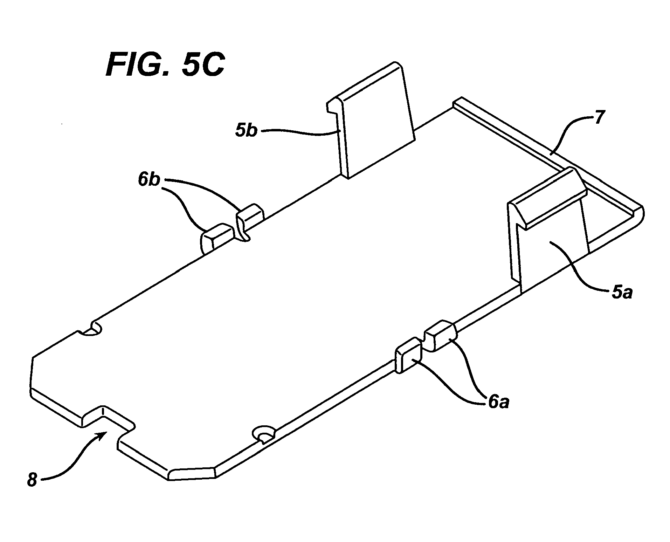

Figure 5C is a perspective view of a floor as seen in Figure 2.

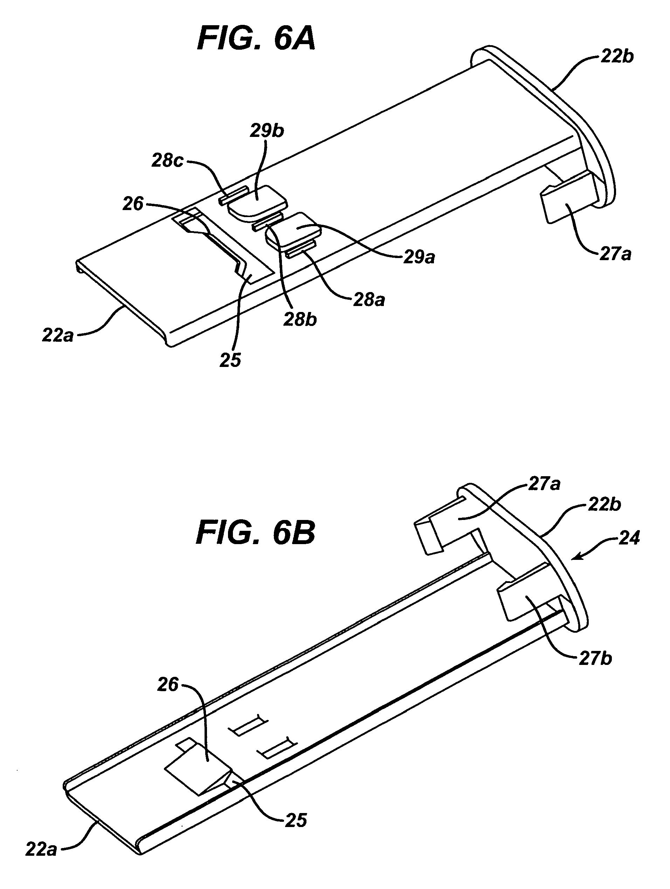

Figure 6A is a top view of a male connector used in devices of the present invention.
Figure 6B is a bottom view of the male connector of Fig. 6A.

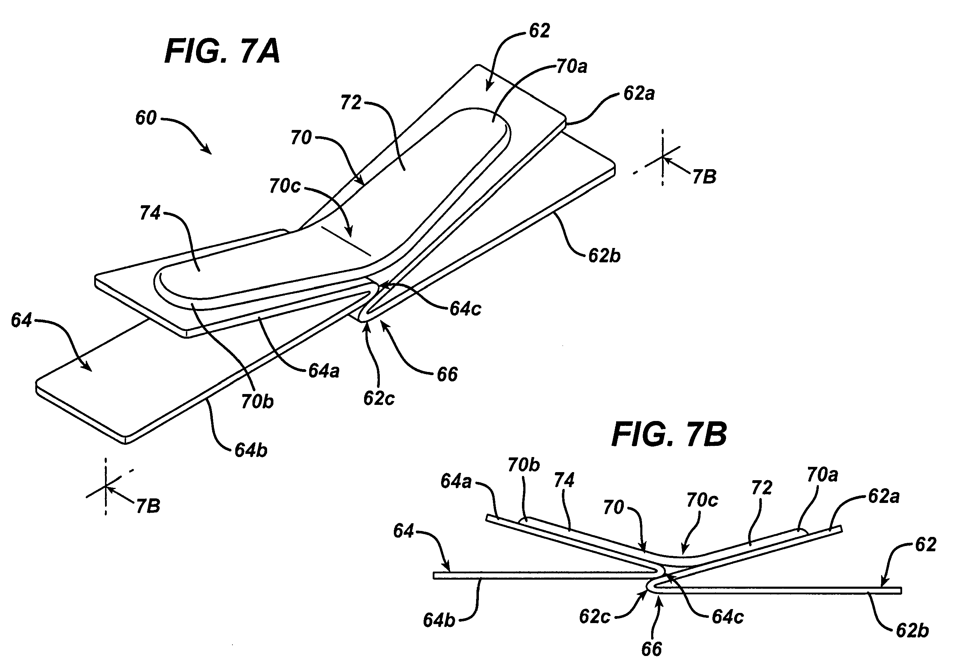

Figure 7A is a perspective view of a personal care product utilized in assemblies and devices of the present inventions.
Figure 7B is a side view of the personal care product shown in Figure 7A.

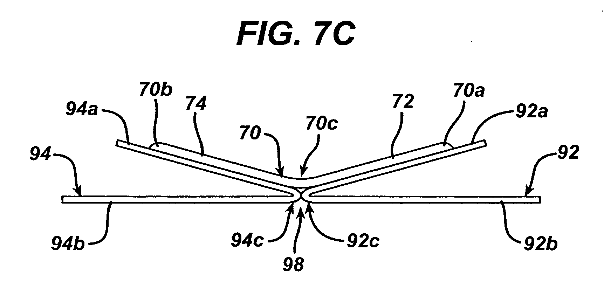

Figure 7C is a side view of an alternate embodiment of a personal care product utilized in assemblies and devices of the present invention;

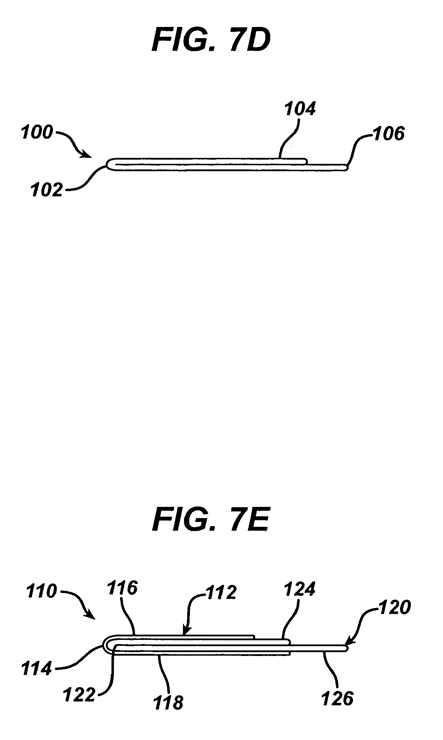

Figure 7D is a side view of an alternate embodiment of a personal care product utilized in assemblies and devices of the present invention.
Figure 7E is a side view of an alternate embodiment of a personal care product utilized in assemblies and devices of the present invention.

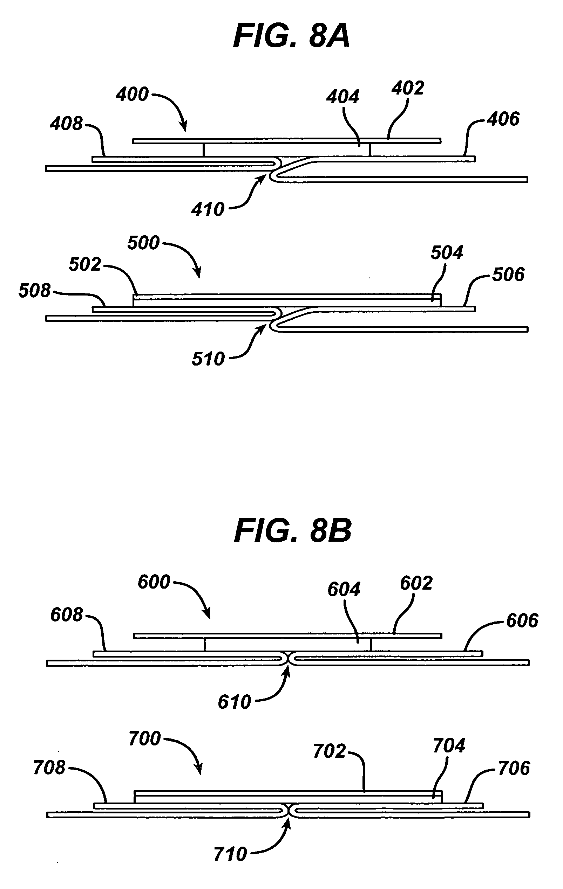

Figure 8A is a side view of personal care products of the present invention having overlapping release liners.
Figure 8B is a side view of alternate embodiments of personal care products of the present invention having abutting release liners.

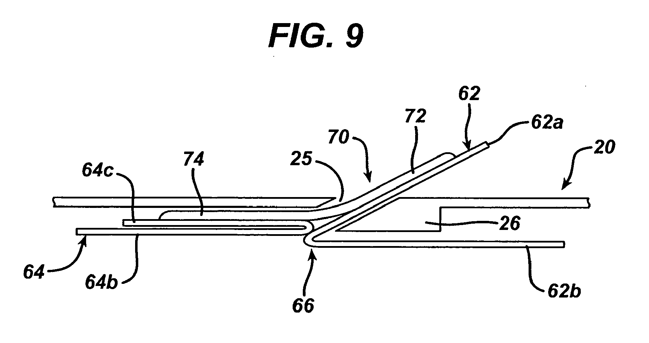

Figure 9 is a side view of the personal care product of 7B in cooperation with the dispensing means of Figures 6A and 6B.

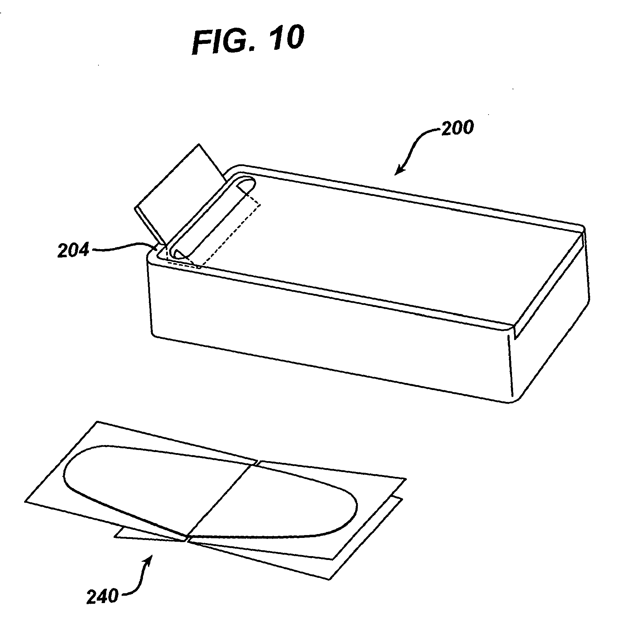

Figure 10 is a top perspective view of an alternate device according to the present invention in a closed position.

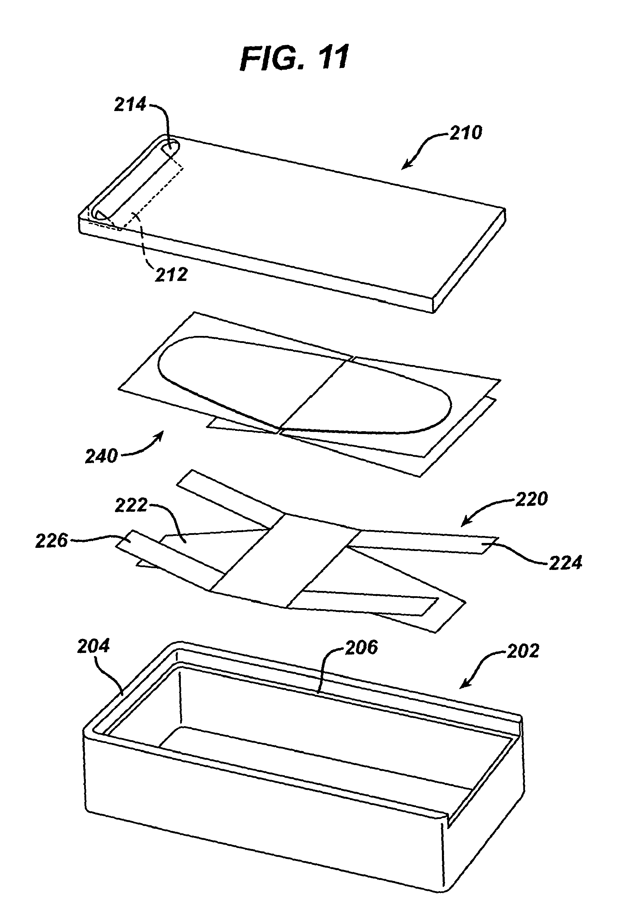

Figure 11 is a perspective view of an unassembled device depicted in Figure 10.

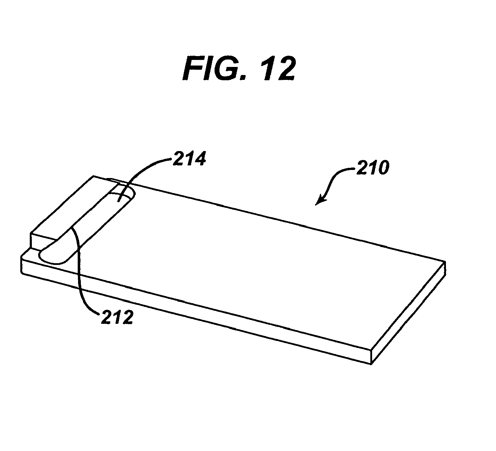

Figure 12 is a perspective bottom view of the dispensing means of the device of Figures 10 and 11.

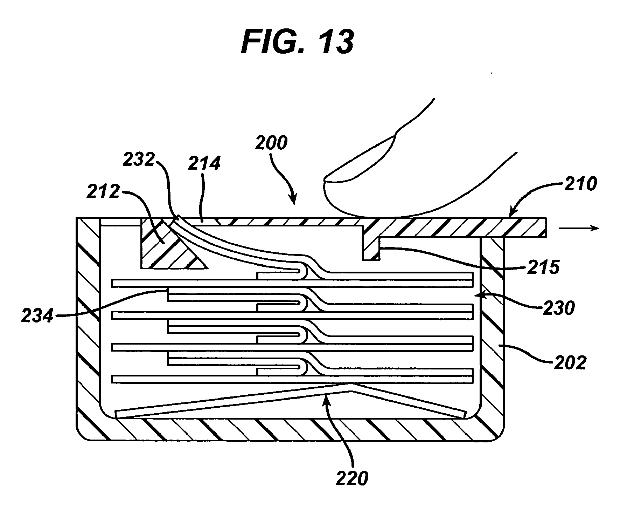

Figure 13 is a side view of the device shown in Figure 10.

### Detailed Description of the Invention

Devices according to the present invention are suitable for both storing and dispensing personal care products from an assembly of such products disposed within the device. Each individual personal care product may be placed one upon the other so as to provide the assembly comprising a plurality of the personal care products, e.g. a stack of products. Once having considered the contents hereof, one skilled in the art will appreciate that the first personal care product may be considered as either the top or the bottom of the assembly, depending on the orientation of the assembly with respect to the storage compartment and the dispensing means.

In certain embodiments, the plurality of personal care products comprises a top personal care product located at the top of the assembly of products, a bottom personal care product located at the bottom of the assembly of products, and multiple personal care products disposed between the top and bottom personal care products. In such cases, the bottom surface of the bottom personal care product may provide a bottom surface of the assembly. Upon operation of the device by sliding, or extending, the storage compartment and the dispensing means longitudinally away from each other from the storage position, where the device is closed, to the dispensing position, where the device is opened, the top personal care product is conveniently exposed through an opening in the dispensing means such that the consumer may easily take hold of and remove the personal care product from the device. The device is then returned to the closed storage position, whereby it is ready to dispense the next personal care product that has advanced to the top of the assembly.

Personal care products useful in the present invention are substantially planar in construction. As used herein, substantially planar means that, while the products are three-dimensional, the length and width are substantially greater than the thickness of the personal care product, such that the product is relatively thin. The product is relatively flexible to the point that it may be manipulated or bent by the dispensing means to expose the top product upon operation of the device, but is relatively rigid as well to prevent the product from binding or sticking within the device. The actual dimensions of the personal care product will vary depending on the particular type of product and the dimensions of the device used to store and dispense the products. Personal care products may have a length of from about 40 to about 60 millimeters, a width of from about 20 to about 30 millimeters, and a thickness of from about 0.2 to about 2 millimeters.

Certain types of personal care products mentioned herein, for example bandages, patches, sanitary napkins, panty liners, or wipes may be contained within individual envelopes, or wrappers, and thus may be isolated one from the other within the bulk packaging of the products. In other embodiments of the present invention, individual personal care products used to form the assemblies utilized in the present invention are free of such individual wrapping in order to take advantage of the improved convenience and efficiencies of such devices. In such embodiments, personal care products used according to the present invention are in direct contact with one another once assembled for placement within the storage compartment and thus are not isolated one from the other by individual wrapping. In certain embodiments, the personal care product is selected from the group consisting of an adhesive bandage, a transdermal patch, a sanitary pad, a pantyliner, and personal wipes, such as cleansing and/or cosmetic wipes. The personal wipes may include and deliver to the skin additives commonly used for cleansing, disinfecting, or providing general cosmetic benefits, for example, soaps, lotions, fragrances, and antibacterial or antimicrobial agents. Transdermal patches may include active agents commonly used and delivered by such patches. The products may be used with or without release liners and may be folded onto themselves where appropriate. Adhesive bandages may include a hydrocolloid bandage comprising a backing layer and an absorbent hydrocolloid layer, with or without an additional absorbent fibrous pad, or bandages having a backing layer and an absorbent pad layer, such as a woven or nonwoven fibrous or non-fibrous pad. The personal care products may be made from conventional materials, including backing materials, adhesives, woven or non-woven substrates or fabrics, and absorbent pads. Conventional release liners, for example, silicone coated kraft paper, polystyrene coated paper, polyethylene sheet release liners and the like, may be utilized where appropriate.

The personal care products suitable for use in the present inventions comprise a first portion extending between a fold line and the distal end of the personal care product, and a second portion extending between the fold line and the proximate end of the personal care product. As used herein the fold line of the personal care product is that point, or area, along the longitudinal axis of the personal care product where the first portion of the product will bend, or fold, in a direction towards the dispensing means when it is contacted thereby. The fold line need not be a physical element of the product itself, such as a pre-formed crease or indentation, although such physical elements are contemplated by the present invention. In certain embodiments the first portion is shorter than the second portion in order to provide more efficient interaction of the product with the dispensing means and thus more efficient dispensing of products from the device. The ratio of the length of the first portion to the second portion may range from about 1:1 to about 1:5, or from about 1:1.1 to about 1:3, or from about 1:1.2 to about 1:2, or from about 1:1.2 to about 1:1.6, or about 1:1.4.

In certain embodiments, the personal care products will comprise at least one release liner adjacent the bottom surface of the product. The release liner is sized and configured such that it may be folded over onto itself at a fold line so as to create a first section extending between the fold line of the liner and the proximal end of the liner and a second section extending between the fold line of the liner and the distal end of the liner. The first section of the liner may extend beyond the edges of the personal care product to which the liner has been applied. The first section will be in contact with the bottom surface of the personal care product. The second section will be folded back upon the first section of the release liner and may extend beyond the end of the first section of the release liner. In certain embodiments the second section of the liner is longer than the first section of the liner. The difference in lengths of the first and second sections provides for more efficient interaction of the product with the dispensing means and thus more efficient dispensing of products from the device. When the personal care product includes such liner, the ratio of the length of the first section to the second section may range from about 1:1 to about 1:5, or from about 1:1.1 to about 1:3, or from about 1:1.2 to about 1:2, or from about 1:1.2 to about 1:1.6, or about 1:1.4.

In other embodiments of the present invention as illustrated herein, two liners may be applied to the bottom surface of, for example, an adhesive bandage or patch. The respective liners are folded onto themselves to form respective first and second sections. The two liners are positioned on the bottom surface of the bandage or patch such that there is an interface area where the respective liners interface one with the other at or about their respective fold lines on the bottom surface of the product. Where the personal care product includes an adhesive layer applied to its bottom surface, the respective liners should be positioned to avoid significant space between the liners at the interface area in order to prevent sticking of the product within the product assembly. Gaps of from about 0.04 inches or greater between the respective liners may cause such sticking. Preferably, the respective liners abut or overlap one another at the interface area. An overlap reduces the likelihood that the products stacked upon each other will stick together and/or bind in the device at the time the products are to be dispensed.

In certain embodiments as illustrated herein the personal care products comprise a backing layer having a top layer and a bottom surface, an absorbent layer affixed and adjacent to at least a first portion of the bottom surface thereof, and two release liners applied adjacent at least a portion of the bottom surface of the backing layer and absorbent layer. The release liners may be positioned such that the liner disposed under the first portion of the backing layer overlaps the liner disposed under the second portion of the backing layer at the interface area of the two liners, as may be seen from a view taken from the side and/or bottom surface of the respective liners. This overlap provides more efficient dispensing of product from the device compared to a product where the liner disposed under the second portion of the backing layer overlaps the liner disposed under the first portion of the backing layer at the interface area. A product using liners constructed to create such an overlap, in combination with an appropriate ratio of the length of the first portion of the product to the second product, is particularly advantageous for use in devices of the present invention.

The thickness of the personal care product at the mid-portion and/or center of the products may be greater than the thickness at the respective end-portions. In one case this may be due to the presence of an absorbable layer or pad located at the mid-portion of the product, for example a bandage. This also may be due to the differences between the respective lengths of the first and second portions of the products themselves, or to the differences in the respective lengths of the first and second sections of the liners. It also may be due to the presence of an overlap of liners about the interface area of the respective liners, as described and illustrated herein. The combination of an absorbable layer or pad and an overlap of liners may provide an even greater difference in the relative thickness of the products at the mid- and end-portions. In certain embodiments, the products may have a thickness of about 1.5 millimeters at the center, about 1.1 millimeters at a mid-portion between the center and end, and a thickness of about 0.05 millimeters at the end. Other embodiments may have consistent thickness across the center and mid-portions of about 0.2 to about 0.3 millimeters, and a thickness of about 0.05 at the end.

Other personal products, such as wipes, etc. may be folded upon themselves to create a short portion and a long portion which would interact with the dispenser as indicated above. There may be multiple folds to obtain the desirable length ratio of first to second portions of personal products to be dispensed. Suitable types of folds include, but are not limited to, c-folds, z-folds and the like.

Assemblies of personal care products according to the present invention may be prepared by arranging the individual products one upon the other so as to provide a vertical assembly of the individual products, e.g. a stack of products, comprising a first product, a second product and multiple other products disposed between the first and second products. The first product will be located proximate the dispensing means so that it may be dispensed thereby when the assembly is disposed in the storage compartment. Where the dispensing means is located at the top of the device, the first product will be the top product. The second product, or bottom product in this case, is located at the bottom of and forms the bottom surface of the assembly. The number of personal care products in such assemblies is not critical and may range from about 5 to about 50, or from about 10 to about 25. Such an assembly permits dispensing of individual products from devices of the present invention.

Due to differences in the construct of certain personal care products as described and illustrated herein, certain assemblies will have a greater thickness at the mid-portion and/or center point than at the end-portions of the assemblies. The difference in height will be multiplied by the total number of products in the assembly. The cumulative effect may be relatively substantial with respect to the scale of the devices used to contain such assemblies and dispense such products.

Assuming an orientation where the dispensing means is located at the top of the device, the devices of the present invention include a compartment for storing the assembly of products slidingly engaged with means for dispensing the top personal care product from the assembly contained within the storage compartment. The storage compartment may comprise a cartridge having distal and proximate ends, a first side wall disposed parallel a longitudenal axis of the cartridge and a second side wall disposed parallel to and spaced apart from the first side wall. A third wall is disposed parallel to a horizontal axis of the cartridge and is disposed transverse to and between the first and second side walls, and a fourth wall is disposed parallel to and spaced apart from the third wall and between the first and second side walls. The configuration and size of the compartment may be determined by the particular product being utilized within the device of the compartment. For instance, the compartment may be in the shape of a parallelogram, e.g. a rectangle or square.

Each of the walls of the cartridge comprise an internal surface proximate the assembly disposed within the cartridge, and an external surface opposite the first surface. The first and second side walls may further comprise at least one projection located on the internal surfaces for maintaining the assembly in a first horizontal position along the horizontal axis. The sidewalls may comprise a single projection of size and configuration effective to maintain the assembly in the proper horizontal position, or may comprise a plurality of projections spaced apart along the longitudinal axis of the compartment.

The third and fourth walls are disposed so as to maintain the assembly in a second longitudinal position along the longitudinal axis. One of the walls may be located at the distal end of the cartridge to form an end piece between the two sidewalls. The other wall may be located near or at the proximate end of the cartridge. The wall located near the proximate end of the cartridge may be adjustable, for example slidable along the longitudinal axis, so as to adapt to different lengths of personal care products utilized in the devices. The distance between the third and fourth walls is effective to maintain the assembly in a proper longitudinal position. The combination of the sidewalls and third and fourth walls serve to maintain the assembly in a third position within the cartridge to effectively and conveniently dispense the top personal care product.

Certain embodiments of devices according to the present invention further comprise means for advancing the plurality of personal care products within the compartment in a vertical direction towards the dispensing means after the first personal care product has been dispensed. Assuming an orientation where the dispensing means is located at the top of the device, the means for advancing may be disposed proximate a bottom surface of the assembly, typically the bottom surface of the bottom personal care product. While the means for advancing may include any mechanism for indexing the products upwards into a position proximate the dispensing means, certain embodiments utilize means for applying pressure to the bottom surface of said assembly. A spring may be used in such embodiments. The spring may be of any design suitable for providing pressure effective to advance the products, for instance coiled springs may be used, or a spring as illustrated in the figures may be used. The spring may be made of plastics or metals, such as stainless steel and the like.

Certain embodiments of devices according to the present invention may comprise means for distributing the pressure substantially equally across the bottom surface of the assembly of personal care products. Equalization of pressure ensures that the products are advanced evenly towards the dispensing means and the assembly is maintained in proper position for dispensing. As a result, the products do not bind when the device is operated to dispense subsequent products.

In certain embodiments, the advancing means itself may be constructed so as to distribute pressure across the assembly as it advances the products towards the dispensing means. The advancing means may comprise a spring member having a mid-point and end-points, where the distance between the base and the mid-point of the advancing member is less than the distance between the base and the end points of the advancing member. In the case where the height at the mid-portion of the product assembly is greater than the height at the end-portion of the product assembly, substantially equal pressure may be applied at the mid and end-portions of the product assembly due to the construct of the advancing member, even though the relative heights of the assembly at the mid and end-portions may be substantially different. The exact difference between the height at the mid and end-portions of the advancing member will depend upon the relative difference between the height at the mid and end-portions of the product assembly. In certain embodiments a semirigid or flexible platform may be employed. When employed, the platform is designed to flex and conform to the varying thickness of the stack as the product is dispensed. The platform is thin enough so that the spring causes it to become flat and to prevent it from taking a 'set' into a concave shape when it is stored for along period of time. This allows the last product to be kept 'flat' by the platform.]

In certain embodiments a relatively rigid platform may be employed. As used herein, relatively rigid means that the platform has sufficient structural strength so as not to be deformed by application of pressure by the advancing means. Such platforms may be disposed between the assembly of products and the means for advancing the products, thus providing substantially equal transfer of pressure from the advancing means across the bottom surface of the assembly. The platform is of a size and configuration that fits within the cartridge walls. It may be of the same size and configuration as the assembly, but it is not necessary for the platform to mirror the assembly, i.e. it may be larger or smaller than the assembly so long as it provides substantially equal distribution of pressure.

The devices may further comprise a floor removably attached to the cartridge where the means for advancing is disposed between the assembly of products and the floor. Removal of the floor, the advancing means and the distribution means allows replacement assemblies to be inserted into the device. Thus, the device may be reused once the entire assembly of products has been dispensed.

The device further comprises a male connector for interacting with the assembly, once it is disposed within the compartment, upon movement from the first closed position to the second open position. The connector comprising a proximate end, a distal end, a protrusion located towards the distal end of the connector for lifting the first portion of the top personal care product, and an opening located proximate and in cooperation with the protrusion for receiving the lifted first portion of the top personal care product upon movement from the first closed position to the second opened position. The protrusion extends downwards from the surface of the male connector at an angle effective to lift the top personal care product from the assembly. The angle may be between about 15 to 70 degrees, or from about 25 to about 60 degrees, or from about 35 to about 50 degrees. The protrusion may be in the shape of a wedge, or any other shape effective to lift the first portion of the top layer product. It may be molded into the body of the male connector, or may be fixedly attached to the bottom surface of the male connector. The first portion of the top layer product is lifted by the protrusion and directed to the opening, for example a slot, thus allowing the first portion of the top product layer to be accessed by the consumer. The opening may be in the shape of a parallelogram, a half-circle or an ovoid, depending on the personal care product being dispensed.

The components of the device of the present invention may be made of any suitable material known in the art and by methods known in the art. For example, the components may be made from polyolefins, such as polyethylene or polypropylene; polyesters, such as polycaprolactone; polyamides, such as nylon; polyvinyl chloride and combinations thereof. The components may be made by injection molding or any other suitable process. The components may be made in unitary construction, or they may be made separately and joined through the use of adhesives, ultrasonic welding, snap fits and the like.

Referring to the Figures, there is shown one device of the present invention for storing and dispensing personal care products. As seen in Figures 1A, 1B and 2, device **50** includes cartridge **1** for storing a plurality of personal care products, each placed directly one upon another to form personal care product assembly, e.g. stack, **40,** and dispensing means **20,** having male connector **22** partially disposed in shell **21.** Cartridge **1** is sized and shaped accordingly to hold assembly **40.**

As seen in Figures 2, 5A, 5B, and 5C, cartridge **1** comprises distal end **80** and proximal end **82,** a storage compartment defined by side walls **2a** and **2b,** wall **2c** located at distal end **80,** and wall **3** located towards proximal end **82,** each wall comprising an internal surface proximate assembly **40** when disposed within the storage compartment, and an external surface opposite the internal surface. Sidewalls **2a** and **2b** may have dovetailed cutouts **13a** and **13b**. The external surface of side walls **2a** and **2b** may have detent bumps **11a** and **11b** (not shown) to create a snap-fit between cartridge **1** and dispensing means **20** when device **50** is in the closed, storage position, and triangular protrusions **10a** and **10b** to provide a stopping feature at the end of a dispensing motion.

The internal surface of side walls **2a** and **2b** may include ribs **12a, 12b, 12c,** and **12d** disposed at spaced-apart intervals along the longitudinal axis of cartridge **1** to maintain assembly **40** in a first substantially centered position with respect to the horizontal axis of cartridge **1** to provide proper alignment of assembly **40** for dispensing top personal care product **42.** Wall **3** is positioned at a point along the longitudinal axis of cartridge **1** to maintain assembly **40** in a second position along the longitudinal axis of cartridge **1** to provide proper alignment of assembly **40** for dispensing top personal care product **42.** The combination of ribs **12a, 12b, 12c,** and **12d** and wall **3** provide alignment of the stack **40** in a third position to facilitate dispensing of top personal care product **42.**

Tabs **9a, 9b, 9c, 9d, 9e,** and **9f** may be utilized to retain assembly **40** in cartridge 1 until each product is individually dispensed. Door snap cutouts **18a** and **18b** help to attach floor **4** to cartridge **1** in a releasable fit. Floor **4** allows for loading and unloading of assembly **40** in cartridge **1.** Floor **4** may have door snaps **5a** and **5b** which mate with door snap cut-outs **18a** and **18b,** and dovetailed forks **6a** and **6b,** which enable the floor to be attached to the walls at dovetailed cut outs **13a** and **13b.** Floor **4** may have a spring lip **7** to keep advancing means **30** from sliding out of cartridge **1.** Floor **4** may also have centering slot **8** to mate with centering tongue **14** to keep the parts properly aligned.

As seen in Figure 2, platform **31** may be disposed between assembly **40** and advancing means **30** to distribute the force applied by advancing means **30** evenly across the bottom surface of assembly **40.** Platform **31** may be made of any suitable material, such as plastic or cardboard, and is sized appropriately to contact the advancing means and to support assembly **40.** Cartridge **1** may have alignment ribs **15a, 15b,** and **15c** and a glue pad **16** for aligning cartridge **1** with cartridge shell **17** and securing cartridge **1** in shell **17.** Cartridge shell **17** may function as a handle or grip for sliding cartridge **1** from the closed storage position to the open dispensing position.

As seen in Figures 2, 3 and 4, advancing means **30** is disposed between platform **31** and floor **4** and includes base **30a,** spring members **30b, 30c** and **30d.** Means **30** applies pressure to the bottom surface of assembly **40** via spring members **30b, 30c** and **30d,** thereby advancing the personal care products in a vertical direction towards dispensing means **20** within cartridge **1,** such that top product **42** in assembly **40** is in a position to be contacted and lifted by dispensing wedge **26** and directed into slot **25** of male connector **22.** The product advancing means may be any suitable material that applies force in the vertical direction. Figure 3 represents advancing means **30** in a fully expanded state, as would be the case in a device prior to loading with assembly **40.** Figure 4 represents advancing means **30** in a compressed state, as would be the case in a device having assembly **40** disposed therein.

As shown, distance **d₁** between base **30a** and the point of spring member **30b** farthest from base **30a** is less than distance **d₂** between base **30a** and the points of spring members **30c** and **30d** farthest from base **30a,** respectively. This construct is particularly advantageous where the thickness, or height, of the product assembly at its mid-point is greater than the thickness, or height, of the product assembly at its respective end-points. As products are dispensed, advancing means **30** expands, resulting in an increase of **d₁** and **d₂**, to maintain substantially equal pressure at the mid and end-points of the assembly and advancing the next product in the assembly into a position to be dispensed.

Referring to Figures 2, 6A and 6B, means for dispensing the personal care products is described. Product-dispensing means **20** contains male connector **22** having proximate end **22a** disposed within dispensing-shell **21.** Male connector **22** includes wedge **26** located adjacent distal end **22a** thereof for separating and lifting first portion **41** of top personal care product **42** from assembly **40** and directing it into dispensing slot **25,** located adjacent protrusion **26.** Slot **25** provides an opening for the consumer to access first portion **41** of the personal care product and pull it out of the cartridge. Cap **24** is adjacent proximate end **22b** of male connector **22.** Cap **24** may have detent arms **27a** and **27b** to engage with detent bumps **11a** and **11b** of the product-containing cartridge. Dispensing-shell **21** may function as a handle or grip for sliding dispensing means **20** during operation of the device. Alignment ribs **28a, 28b,** and **28c** and glue pads **29a** and **29b** may be utilized to align and secure male connector **22** within dispensing-shell **21.**

Referring to Figures 7A-7C, where like numerals refer to like features of the drawings, bandages that may be utilized in personal care product assemblies of the present invention are described. Bandage **60** includes adhesive bandage **70** having release liners **62** and **64** affixed to the bottom surface thereof. Adhesive bandage **70** comprises an absorbent layer (not shown) and an adhesive layer (not shown) disposed on its bottom surface. Bandage **70** includes first portion **72,** second portion **74,** fold line **70c,** proximal end **70b** and distal end **70a.** As seen in Figures **7A** and **7B****,** release liner **64** is placed under second portion **74** and folded back onto itself at fold line **64c** and creates first liner section 64a and second liner section 64b. Release liner **62** is placed under first portion **72** and folded back onto itself at fold line **62c** to create first liner section **62a** and second liner section **62b.** Release liner **62** overlaps release liner **64** at interface area **66.** As seen in Figure **7C****,** release liners **92** and **94** are folded at fold lines **92c** and **94c** and positioned such that there is no overlap of the liners **92** and **94** at interface area **98.**

Alternate embodiments of personal care products of the present invention are seen in Figures 7D and 7E. In figure 7D, personal care product **100** is folded onto itself at fold line **102** to provide first portion **104** and second portion **106.** In Figure 7E, personal care product **110** includes bandage **112** folded onto itself at fold line **114** to provide first portion **116** and second portion **118** and liner **120** folder onto itself at fold line **122** to provide first liner section **124** and second liner section **126.**

In Figure 8, dispensing means **20** is shown in a dispensing position with a personal care product as shown in Figures 7A and 7B. Bandage **60** includes adhesive bandage **70** having first and second portions **72** and **74** respectively, liner **62** having first and second sections **62a** and **62b,** respectively, and liner **64** having first and second sections **64a** and **64b,** respectively. Liner **62** overlaps liner **64** at interface area **66.** Upon operation of the device, wedge **26** of dispensing means **20** contacts liner **62** and lifts liner section **62a,** and thus first portion **72** of adhesive bandage **70** affixed thereto, and directs liner section **62a** and first portion **72** into slot **25,** thus providing access of bandage **60** by the consumer. The combination of features of liner section **62a** being longer than **62b** and the overlap of liner **62** over liner **64** at interface area **66** serve to provide improved dispensing of products from the device.

Personal care products of the present invention are illustrated in Figures 8A and 8B. In Figure 8A, bandage **400** includes backing layer **402,** non-woven absorbent layer **404,** and release liners **406** and **408.** Release liners **406** and **408** overlap at interface area **410.** Bandage **500** includes backing layer **502,** hydrocolloid absorbent layer **504,** and release liners **506** and **508.** Release liners **506** and **508** overlap at interface area **510.** In Figure 8B, bandage **600** includes backing layer **602,** non-woven absorbent layer **604,** and release liners **606** and **608.** Release liners **606** and **608** overlap at interface area **610.** Bandage **700** includes backing layer **702,** hydrocolloid absorbent layer **704,** and release liners **706** and **708.** Release liners **706** and **708** overlap at interface area **710.**

An alternate embodiment of the invention is shown in Figures 10-13, where like numerals refer to like elements. Device **200** includes storage compartment **202,** dispensing means **210** and advancing means **220.** Storage compartment **202** includes end-stop **204** and recessed track **206** for receiving dispensing means **210** in sliding engagement. Dispensing means **210** includes contact member **212** for contacting and directing the personal care product and slot **214** for receiving the personal care product. Dispensing means **210** also may include stopping member **215** to contact end-stop **204,** thereby limiting the travel of dispensing means **210.** Advancing means **220** includes spring members **222, 224** and **226,** respectively. Figure 10 shows product **240** and device **200.** Figure 13 illustrates the operation of device **200** with product assembly **230** disposed therein. As dispensing means **210** is moved in the direction of the arrow, contact member **212** contacts and lifts a portion of personal care product **232** and directs product **232** into slot **214.** After removal of product **232** and upon returning advancing means **210** to the closed storage position, advancing member **220** advances assembly **230** in a vertical direction towards dispensing means **220** for subsequent dispensing of the next personal care product **234,** and so on, until all product has been dispensed.

### Example 1

The following example is illustrative of devices according to the present invention. The claims should not be construed to be limited to the details thereof.

The device depicted in the Figures was made by a streolithography additive process. Adhesive bandages according to Figures 7A and 7B were hand made utilizing conventional adhesive bandage backing materials, adhesives, wound contacting pads, and release liners. The bandages were stacked one upon the other to form an assembly of the bandages as represented in Figures 1B and 2 and then placed in the cartridge with the first short portions adjacent the distal end of the cartridge. The platform was placed on the spring. The spring and the platform were placed on the floor and the three parts attached to the cartridge via a snap fit between the floor and the cartridge. The proximate end of the cartridge was placed within the shell and the two were glued together. The distal end of the connector was placed within the product dispensing shell and the two were glued together. The distal end of the cartridge was then placed into the product-dispensing shell and advanced forward in a sliding motion to a first closed position. The cartridge and dispensing means were then pulled apart in a sliding motion to a second open position, at which time the first portion of the adhesive bandage was directed into the dispensing slot by the dispensing wedge. The bandage was then removed from the device and the cartridge and dispensing means were pushed back together in a sliding motion to return to the first closed position.

## Claims

1. A device suitable for storing and dispensing personal care products, each personal care product of which is placed one upon the other so as to provide an assembly comprising a plurality of said personal care products, said plurality of personal care products comprising a first personal care product, a second personal care product and multiple other personal care products disposed between said first and second personal care products, said device comprising:
a compartment for storing said assembly; and
means for dispensing said personal care products, said dispensing means comprising a member for contacting a first portion of said first personal care product and an opening for receiving said contacted first portion of said first personal care product,
wherein said compartment and said dispensing means are slidingly engaged between a first storage position and a second dispensing position, and wherein when said assembly is disposed within said compartment and upon movement of said device from said first storage position to said second dispensing position, said first portion of said first personal care product is accessed through said opening.

2. The device of claim 1 further comprising means for advancing said plurality of personal care products in a vertical direction towards said dispensing means after said first personal care product has been dispensed, said advancing means disposed proximate a bottom surface of said assembly.

3. The device of claim 2 wherein said advancing means comprises means for applying pressure to said bottom surface of said assembly once said assembly is disposed within said compartment.

4. The device of claim 3 further comprising means for distributing said pressure substantially equally across said bottom surface of said assembly, said distributing means disposed between said assembly and said advancing means.

5. The device of claim 2 wherein said compartment comprises a cartridge having a first side wall disposed parallel to a longitudenal axis of said cartridge and a second side wall disposed parallel to and spaced apart from said first side wall, a third wall disposed parallel to a horizontal axis of said cartridge transverse to and between said first and second side walls, and a fourth wall disposed parallel to and spaced apart from said third wall and between said first and second side walls, each of said walls comprising an internal surface proximate said assembly once disposed within said cartridge, and an external surface opposite said first surface.

6. The device of claim 5 further comprising a floor removably attached to said cartridge, said advancing means disposed between said assembly and said floor.

7. The device of claim 6 wherein said first and second side walls further comprise a projection disposed on said internal surfaces thereof for maintaining said assembly in a first horizontal position, and said third and fourth walls are disposed along said longitudinal axis so as to maintain said assembly in a second longitudinal position, whereby said assembly is maintained in a third position within said cartridge for dispensing said first personal care product.

8. The device of claim 3 wherein said advancing means comprises a spring.

9. The device of claim 4 wherein said advancing means comprises a spring and said distributing means comprises a platform.

10. The device of claim 8 wherein said compartment comprises a cartridge having a first side wall disposed parallel to a longitudenal axis of said cartridge and a second side wall disposed parallel to and spaced apart from said first side wall, a third wall disposed parallel to a horizontal axis of said cartridge transverse to and between said first and second side walls, and a fourth wall disposed parallel to and spaced apart from said third wall and between said first and second side walls, each of said walls comprising an internal surface proximate said assembly once disposed within said cartridge, and an external surface opposite said first surface.

11. The device of claim 10 further comprising a floor removabley attached to said cartridge, said spring disposed between said distributing means and said floor.

12. The device of claim 11 wherein said first and second side walls further comprise a projection disposed on said internal surfaces thereof for maintaining said assembly in a first horizontal position, and said third and fourth walls are disposed along said longitudinal axis so as to maintain said assembly in a second longitudinal position, whereby said assembly is maintained in a third position within said cartridge for dispensing said first personal care product.

13. The device of claim 1 wherein said dispensing means comprises a male connector for interacting with said assembly once disposed within said compartment upon movement from said first storage position to said second dispensing position, said connector comprising a proximate end and a distal end, said member for contacting said first portion of said first personal care product located towards said distal end, and said opening for receiving said contacted first portion of said first personal care product located proximate said contacting member.

14. The device of claim 5 wherein said dispensing means comprises a male connector for interacting with said assembly once disposed within said cartridge upon movement from said first storage position to said second dispensing position, said connector comprising a proximate end and a distal end, said member for contacting said first portion of said first personal care product located towards said distal end, and said opening for receiving said contacted first portion of said first personal care product located proximate said contacting member.

15. The device of claim 2 wherein said dispensing means comprises a male connector for interacting with said assembly once disposed within said cartridge upon movement from said first storage position to said second dispensing position, said connector comprising a proximate end and a distal end, said member for contacting said first portion of said first personal care product located towards said distal end, and said opening for receiving said contacted first portion of said first personal care product located proximate said contacting member.

16. The device of claim 15 wherein said advancing means comprises means for applying pressure to said bottom surface of said assembly once said assembly is disposed within said compartment.

17. The device of claim 16 further comprising means for distributing said pressure substantially equally across said bottom surface of said assembly, said distributing means disposed between said assembly and said advancing means.

18. The device of claim 13 further comprising a housing having said proximate end of said male connector disposed therein.

19. The device of claim 15 further comprising a floor removably attached to said cartridge, said advancing means disposed between said assembly and said floor.

20. The device of claim 3 wherein said advancing means comprises a spring.

21. The device of claim 17 wherein said advancing means comprises a spring and said distributing means comprises a platform.

22. The device of claim 1 further comprising said assembly disposed within said compartment.

23. The device of claim 5 further comprising said assembly disposed within said cartridge.

24. The device of claim 10 further comprising said assembly disposed within said cartridge.

25. The device of claim 13 further comprising said assembly disposed within said compartment.
